# EUROPEAN PATENT SPECIFICATION

(11) **EP 2 491 017 B1**
(45) Date of publication and mention of the grant of the patent: **27.08.2014**
(21) Application number: 10769130.5
(22) Date of filing: 20.10.2010
(51) Int. Cl.: C07D 207/22, A61K 31/40, A61P 29/00, A61P 25/18, A61P 17/06

(54) **ALPHA ADRENERGIC RECEPTOR MODULATORS**
ALPHA-ADRENERGISCHE REZEPTORMODULATOREN
MODULATEURS DU RÉCEPTEUR ALPHA-ADRÉNERGIQUE

(30) Priority: 21.10.2009 US 253654 P
(43) Date of publication of application: 29.08.2012
(73) Proprietor: ALLERGAN, INC., Irvine, CA 92612 (US)
(72) Inventor: CHOW, Ken, Newport Coast California 92657 (US); WANG, Liming, Irvine California 92612 (US); FANG, Wenkui, K., Irvine California 92618 (US); CORPUZ, Evelyn, G., Irvine California 92602 (US); GIL, Daniel, W., Corona Del Mar California 92625 (US)
(74) Representative: HOFFMANN EITLE
(86) International application number: PCT/US2010/053327
(87) International publication number: WO 2011/050030

(56) References cited:
- MONTSENIGOS A ET AL: "Alpha adrenergic clonidine derivatives. QSAR by MTD for multiconformational molecules" REVUE ROUMAINE DE CHIMIE - ROMANIAN JOURNAL OF CHEMISTRY, vol. 34, no. 11-12, 1 January 1989 (1989-01-01), pages 2101-2106, XP009144318 ISSN: 0035-3930 cited in the application
- HERSHENSON, F.M.; ET AL.: "Synthesis and Antihypertensive Activity of 2-Arylamino-1-azacycloalkenes" JOURNAL OF MEDICINAL CHEMISTRY, vol. 14, no. 10, 1971, pages 907-909, XP002622490

## Description

### Background

Human adrenergic receptors are integral membrane proteins which have been classified into two broad classes, alpha and the beta adrenergic receptors. Both types of receptors mediate the action of the peripheral sympathetic nervous system upon binding of catecholamines, norepinephrine and epinephrine.

Norepinephrine is produced by adrenergic nerve endings, while epinephrine is produced by the adrenal medulla. The binding affinity of adrenergic receptors for these compounds forms one basis of classification: alpha receptors tend to bind norepinephrine more strongly than epinephrine. The preferred binding affinity of these hormones is reversed for the beta receptors. In many tissues, the functional responses, such as smooth muscle contraction, induced by alpha receptor activation are opposed to responses induced by beta receptor binding.

The functional distinction between alpha and beta receptors is further highlighted and refined by the pharmacological characterization of these receptors from various animal and tissue sources. Alpha and beta adrenergic receptors are further subdivided into alpha-1 (α₁), alpha-2 (α₂), beta-1 (β₁), and beta-2 (β₂) subtypes.

Functional differences between alpha-1 and alpha-2 receptors have been recognized, and compounds which exhibit selective binding between these two subtypes have been developed. Thus, the selective ability of the R(+) enantiomer of terazosin to selectively bind to adrenergic receptors of the alpha-1 subtype has been reported. The alpha-1 / alpha-2 selectivity of this compound is conventionally known as being significant because agonist stimulation of the alpha-2 receptor is said to inhibit secretion of epinephrine and norepinephrine, while antagonism of the alpha-2 receptor is said to increase secretion of these hormones. Thus, the use of non-selective alpha-adrenergic blockers, such as phenoxybenzamine and phentolamine, is said to be limited by their alpha-2 adrenergic receptor mediated induction of increased plasma catecholamine concentration and the attendant physiological sequelae (increased heart rate and smooth muscle contraction).

The cloning, sequencing and expression of alpha receptor subtypes from animal tissues has led to the subclassification of the alpha-1 adrenoreceptors into alpha-1A, alpha-1B, and alpha-1D. Similarly, the alpha-2 adrenoreceptors have also been classified: alpha-2A, alpha-2B, and alpha-2C receptors. Each alpha-2 receptor subtype appears to exhibit its own pharmacological and tissue specificities. Compounds having a degree of specificity for one or more of these subtypes may be more effective therapeutic agents for a given indication than an alpha-2 receptor pan-agonist (such as the drug clonidine) or a pan-antagonist.

### Summary

The present invention relates generally to certain heterocyclic compounds and to their use as alpha adrenergic receptor modulators. These compounds are useful in treating a wide variety of disorders associated with modulation of alpha adrenergic receptors. Further, these compounds are useful for the treatment of humans with diseases and conditions that are alleviated by alpha adrenergic modulation, and in particular, useful as alpha-2C modulators. Compositions are also described herein comprising a pharmaceutically acceptable amount of a compound as described herein. Further described are methods for treating conditions using the compositions described herein, wherein the conditions are selected from the group consisting of including but not limited to treating glaucoma, elevated intraocular pressure, ischemic neuropathies, optic neuropathy, pain, visceral pain, corneal pain, headache pain, migraine, cancer pain, back pain, irritable bowel syndrome pain, muscle pain and pain associated with diabetic neuropathy, the treatment of diabetic retinopathy, other retinal degenerative conditions, stroke, schizophrenia, cognitive deficits, neuropsychiatric conditions, drug dependence and addiction, withdrawal symptoms, obsessive-compulsive disorders, obesity, insulin resistance, stress-related conditions, diarrhea, diuresis, nasal congestion, spasticity, attention deficit disorder, psychoses, anxiety, depression, stress urinary incontinence, dilation of the pupil, increase blood pressure, treating nasal congestion, vasoconstriction, cardiac ischemia, autoimmune disease, Crohn's disease, gastritis, Alzheimer's, Parkinson's ALS, and other neurodegenerative diseases, dermatological conditions, skin erythema (redness) and inflammation, rosacea, acne, psoriasis, inflammatory bowel disease (IBD), post-traumatic stress disorder (PTSD), Tourette's syndrome, multiple sclerosis, dry eye disease or combinations thereof.

In one embodiment of the invention there are provided novel compounds that are useful as alpha adrenergic receptor modulators. These compounds are useful in treating a wide variety of disorders associated with modulation of alpha adrenergic receptors. Further, these compounds are useful for the treatment of humans with diseases and conditions that are alleviated by alpha adrenergic modulation, and in particular, useful as alpha-2C modulators. The compounds described herein are N-(2,3-substituted phenyl)-3,4-dihydro-2H-pyrrol-5-amine derivatives.

In another embodiment of the invention, there are provided methods for treating a disorder associated with modulation of the alpha 2C adrenergic receptor. Such methods can be performed, for example, by administering to a subject in need thereof a pharmaceutical composition containing a therapeutically effective amount of at least one compound of Formula I.

In another embodiment of the invention, there are provided methods for treating a disorder associated with modulation of the alpha 2C adrenergic receptor by administering to a subject in need thereof a pharmaceutical composition containing a therapeutically effective amount of at least one compound selected from:
N-(2,6-dichlorophenyl)-3,4-dihydro-2H-pyrrol-5-amine;
N-(2-methylphenyl)-3,4-dihydro-2H-pyrrol-5-amine;
N-[2-(trifluoromethyl)phenyl]-3,4-dihydro-2H-pyrrol-5-amine;
N-(2,6-dimethylphenyl)-3,4-dihydro-2H-pyrrol-5-amine;
N-(2,3-dimethylphenyl)-3,4-dihydro-2H-pyrrol-5-amine.

Further, pharmaceutically acceptable salts, hydrates, solvates, crystal forms and individual isomers, enantiomers, diastereomers and prodrugs of the compounds described herein can be utilized.

Compounds:
N-(2-chlorophenyl)-3,4-dihydro-2H-pyrrol-5-amine;
N-(2,6-dichlorophenyl)-3,4-dihydro-2H-pyrrol-5-amine;
N-(2-methylphenyl)-3,4-dihydro-2H-pyrrol-5-amine;
N-[2-(trifluoromethyl)phenyl]-3,4-dihydro-2H-pyrrol-5-amine;
N-(2,6-dimethylphenyl)-3,4-dihydro-2H-pyrrol-5-amine; and
N-(2,3-dimethylphenyl)-3,4-dihydro-2H-pyrrol-5-amine
are described in:
Journal of American Chemical Society 1975, Vol 97 pages 2811-2818, as cyclic amidines for a tautomeric NMR study;
Journal of Medicinal Chemistry (1975), 18(1), 90-9, as amidines and related
compounds in "Structure-activity relations of antihypertensive and antisecretory agents related to clonidine";
Patent ZA68022962 (1968), as (o-substituted-phenylimino)-1- aza cycloalkanes;
Revue Roumaine de Chimie (1989), 34(11-12), 2101-6, as alpha adrenergic clonidine derivatives.

### Detailed Description

In one embodiment, the invention therefore provides a compound having Formula I, 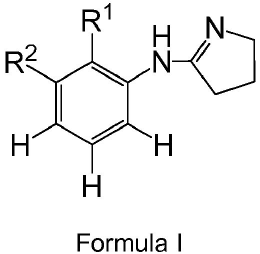 wherein:
R¹ is selected from methoxy, methyl, chloro, fluoro, bromo; and
R² is selected from methoxy, methyl, chloro, fluoro, bromo, with the proviso that R¹ and R² cannot be methyl in same time.

In another embodiment described herein is a method for treating a disorder associated with selective subtype modulation of the alpha 2C adrenergic receptor, comprising administering to a subject in need thereof a pharmaceutical composition containing a therapeutically effective amount of at least one compound selected from the group consisting of:
N-(2-chlorophenyl)-3,4-dihydro-2H-pyrrol-5-amine;
N-(2,6-dichlorophenyl)-3,4-dihydro-2H-pyrrol-5-amine;
N-(2-methylphenyl)-3,4-dihydro-2H-pyrrol-5-amine;
N-[2-(trifluoromethyl)phenyl]-3,4-dihydro-2H-pyrrol-5-amine;
N-(2,6-dimethylphenyl)-3,4-dihydro-2H-pyrrol-5-amine;
N-(2,3-dimethylphenyl)-3,4-dihydro-2H-pyrrol-5-amine;
N-(2-bromo-3-methyl-phenyl)-3,4-dihydro-2H-pyrrol-5-amine;
N-(3-chloro-2-methoxyphenyl)-3,4-dihydro-2H-pyrrol-5-amine;
N-(3-chloro-2-methylphenyl)-3,4-dihydro-2H-pyrrol-5-amine;
N-(2-chloro-3-methylphenyl)-3,4-dihydro-2H-pyrrol-5-amine;
N-(2-methoxy-3-methylphenyl)-3,4-dihydro-2H-pyrrol-5-amine;
N-(3-chloro-2-fluorophenyl)-3,4-dihydro-2H-pyrrol-5-amine;
N-(2-bromo-3-methoxyphenyl)-3,4-dihydro-2H-pyrrol-5-amine;
N-(3-methoxy-2-methylphenyl)-3,4-dihydro-2H-pyrrol-5-amine;
N-(3-fluoro-2-methylphenyl)-3,4-dihydro-2H-pyrrol-5-amine;
N-(3-bromo-2-methylphenyl)-3,4-dihydro-2H-pyrrol-5-amine.

Further, pharmaceutically acceptable salts, hydrates, solvates, crystal forms and individual isomers, enantiomers, diastereomers and prodrugs of these compounds can be utilized.

As used herein, "pharmaceutically acceptable salt" refers to any salt that retains the activity of the parent compound and does not impart any additional deleterious or untoward effects on the subject to which it is administered and in the context in which it is administered compared to the parent compound. A pharmaceutically acceptable salt also refers to any salt which may form *in vivo* as a result of administration of an acid, another salt, or a prodrug which is converted into an acid or salt. Further,"pharmaceutically acceptable salt " refers to those salts which retain the biological effectiveness and properties of the free bases and which are obtained by reaction with acids such as but not limited to: hydrochloric acid, hydrobromic acid, fumaric acid, sulfuric acid, nitric acid, phosphoric acid, methanesulfonic acid, ethanesulfonic acid, p-toluenesulfonic acid, salicylic acid and the like.

Pharmaceutically acceptable salts of acidic functional groups may be derived from organic or inorganic bases. The salt may comprise a mono or polyvalent ion. Of particular interest are the inorganic ions, lithium, sodium, potassium, calcium, and magnesium. Organic salts may be made with amines, particularly ammonium salts such as mono-, di- and trialkyl amines or ethanol amines. Salts may also be formed with caffeine, tromethamine and similar molecules. Hydrochloric acid or some other pharmaceutically acceptable acid, may form a salt with a compound that includes a basic group, such as an amine or a pyridine ring.

As used herein "prodrug" refers to a compound which is converted to a therapeutically active compound after administration, and the term should be interpreted as broadly herein as is generally understood in the art. While not intending to limit the scope of the present description, conversion may occur by hydrolysis of an ester group or some other biologically labile group. Generally, but not necessarily, a prodrug is inactive or less active than the therapeutically active compound to which it is converted. Ester prodrugs of the compounds disclosed herein are contemplated. An ester may be derived from a carboxylic acid of C₁ (i.e. the terminal carboxylic acid of a natural prostaglandin), or an ester may be derived from a carboxylic acid functional group on another part of the molecule, such as on a phenyl ring. While not intending to be limiting, an ester may be an alkyl ester, an aryl ester, or a heteroaryl ester.

As used herein, "tautomer" refers to the migration of protons between adjacent single and double bonds. The tautomerization process is reversible. Compounds described herein can undergo any possible tautomerization that is within the physical characteristics of the compound. The following is an example of tautomerization that can occur in compounds described herein:

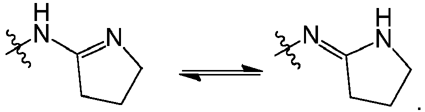

As used herein, the term "therapeutically effective amount" means the amount of the pharmaceutical composition that will elicit the biological or medical response of a subject in need thereof that is being sought by the researcher, veterinarian, medical doctor or other clinician. In some embodiments, the subject in need thereof is a mammal. In some embodiments, the mammal is human.

It is to be understood that both the foregoing detailed description is exemplary and explanatory only and are not restrictive. As used herein, the use of the singular includes the plural unless specifically stated otherwise. As used herein, "or" means "and/or" unless stated otherwise. Furthermore, use of the term "including" as well as other forms, such as "includes," and "included," is not limiting. The section headings used herein are for organizational purposes only and are not to be construed as limiting the subject matter described.

Unless specific definitions are provided, the nomenclature utilized in connection with, and the laboratory procedures and techniques of analytical chemistry, synthetic organic and inorganic chemistry described herein are those known in the art. Standard chemical symbols are used interchangeably with the full names represented by such symbols. Thus, for example, the terms "methyl" and "CH₃" are understood to have identical meaning. Standard techniques may be used for chemical syntheses, chemical analyses, and formulation. The structures depicted and described herein are intended to include every possible stereoisomer/tautomer, both pure or in any possible mixture.

Unless otherwise indicated, all numbers expressing quantities of ingredients, properties such as molecular weight, reaction conditions, and so forth used in the specification and claims are to be understood as being modified in all instances by the term "about." Accordingly, unless indicated to the contrary, the numerical parameters set forth in the specification and attached claims are approximations that may vary depending upon the desired properties sought to be obtained by the present invention. At the very least, and not as an attempt to limit the application of the doctrine of equivalents to the scope of the claims, each numerical parameter should at least be construed in light of the number of reported significant digits and by applying ordinary rounding techniques. Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard deviation found in their respective testing measurements.

Specific embodiments disclosed herein may be further limited in the claims using consisting of or/and consisting essentially of language. When used in the claims, whether as filed or added per amendment, the transition term "consisting of" excludes any element, step, or ingredient not specified in the claims. The transition term "consisting essentially of" limits the scope of a claim to the specified materials or steps and those that do not materially affect the basic and novel characteristic(s). Embodiments of the invention so claimed are inherently or expressly described and enabled herein.

The terms "a," "an," "the" and similar referents used in the context of describing the invention (especially in the context of the following claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein is intended merely to better illuminate the invention and does not pose a limitation on the scope of the invention otherwise claimed. No language in the specification should be construed as indicating any non-claimed element essential to the practice of the invention.

Groupings of alternative elements or embodiments of the invention disclosed herein are not to be construed as limitations. Each group member may be referred to and claimed individually or in any combination with other members of the group or other elements found herein. It is anticipated that one or more members of a group may be included in, or deleted from, a group for reasons of convenience and/or patentability. Certain embodiments are described herein, including the best mode known to the inventors for carrying out the invention. Of course, variations on these described embodiments will become apparent to those of ordinary skill in the art upon reading the foregoing description. The inventor expects skilled artisans to employ such variations as appropriate, and the inventors intend for the invention to be practiced otherwise than specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

It is to be understood that the embodiments disclosed herein are illustrative of the principles of the present invention. Other modifications that may be employed are within the scope of the invention. Thus, by way of example, but not of limitation, alternative configurations of the present invention may be utilized in accordance with the teachings herein. Accordingly, the present invention is not limited to that precisely as shown and described.

The compounds described herein are useful as medicaments, or in compositions or formulations in mammals, including humans, for treatment of diseases and or alleviations of conditions which are responsive to treatment by modulation of alpha adrenergic receptors. Thus, in further example embodiments, there are provided methods for treating a disorder associated with modulation of alpha adrenergic receptors. Such methods can be performed, for example, by administering to a subject in need thereof a pharmaceutical composition containing a therapeutically effective amount of at least one compound described herein.

The conditions and diseases treatable using the compounds described herein include, but are not limited to, glaucoma, elevated intraocular pressure, ischemic neuropathies, optic neuropathy, pain, visceral pain, corneal pain, headache pain, migraine, cancer pain, back pain, irritable bowel syndrome pain, muscle pain and pain associated with diabetic neuropathy, the treatment of diabetic retinopathy, other retinal degenerative conditions, stroke, cognitive deficits, neuropsychiatric conditions, drug dependence and addiction, withdrawal symptoms, obsessive-compulsive disorders, obesity, insulin resistance, stress-related conditions, diarrhea, diuresis, nasal congestion, spasticity, attention deficit disorder, psychoses, anxiety, depression, autoimmune disease, Crohn's disease, gastritis, Alzheimer's, Parkinson's ALS, and other neurodegenerative diseases, dermatological conditions, skin erythema (redness) and inflammation, rosacea, acne, psoriasis, inflammatory bowel disease (IBD), post-traumatic stress disorder (PTSD), Tourette's syndrome, multiple sclerosis, dry eye disease or combinations thereof.

As mentioned, the compounds described herein are useful in the treatment of chronic pain. By "chronic pain" is meant pain other than acute pain, such as, without limitation, neuropathic pain, visceral pain (including that brought about by Crohn's disease and irritable bowel syndrome (IBS)), and allodynia.

The compounds described herein may be administered at pharmaceutically effective dosages. Such dosages are normally the minimum dose necessary to achieve the desired therapeutic effect. Generally, such doses will be in the range of about 1 mg/day to about 1000 mg/day; more preferably in the range of about 10 mg/day to about 500 mg/day. In another example embodiment, the compound or compounds may be present in a composition or formulation in a range of about 0.5 mg/kg/day to about 100 mg/kg/day or about 1 mg/kg/day to about 100 mg/kg/day. However, the actual amount of the compound to be administered in any given case will be determined by a physician taking into account the relevant circumstances, such as the severity of the pain, the age and weight of the patient, the patient's general physical condition, the cause of pain, and the route of administration.

In another example embodiment, provided are pharmaceutical compositions including at least one compound in a pharmaceutically acceptable carrier. Pharmaceutical compositions can be used in the form of a solid, a solution, an emulsion, a dispersion, a micelle, a liposome, and the like, wherein the resulting composition contains one or more compounds described herein, as active ingredient, in admixture with an organic or inorganic carrier or excipient suitable for enteral or parenteral applications. One or more compounds may be combined, for example, with the usual non-toxic, pharmaceutically acceptable carriers for tablets, pellets, capsules, suppositories, solutions, emulsions, suspensions, and any other form, suitable for use. The carriers which can be used include glucose, lactose, gum acacia, gelatin, mannitol, starch paste, magnesium trisilicate, talc, corn starch, keratin, colloidal silica, potato starch, urea, medium chain length triglycerides, dextrans, and other carriers suitable for use in manufacturing preparations, in solid, semisolid, or liquid form. In addition auxiliary, stabilizing, thickening and coloring agents and perfumes may be used. Compounds described herein are included in pharmaceutical compositions in an amounts sufficient to produce the desired effect upon the process or disease condition.

In one example embodiment, the compounds described herein can be administered orally in any acceptable form, such as a tablet, liquid, capsule, powder and the like. However, other routes may be desirable or necessary, particularly if the patient suffers from nausea. Such other routes may include, without exception, transdermal, parenteral, subcutaneous, intranasal, intrathecal, intramuscular, intravenous, and intrarectal modes of delivery. Additionally, formulations may be designed to delay release of the active compound over a given period of time, or to carefully control the amount of drug released at a given time during the course of therapy.

Pharmaceutical compositions in a form suitable for oral use, for example, are administered as tablets, troches, lozenges, aqueous or oily suspensions, dispersible powders or granules, emulsions, hard or soft capsules, or syrups or elixirs. Compositions intended for oral use may be prepared according to any method known to the art for the manufacture of pharmaceutical compositions and such compositions may contain one or more agents selected from the group consisting of a sweetening agent such as sucrose, lactose, or saccharin, flavoring agents such as peppermint, oil of wintergreen or cherry, coloring agents and preserving agents in order to provide pharmaceutically elegant and palatable preparations. Tablets containing compounds described herein in admixture with non-toxic pharmaceutically acceptable excipients may also be manufactured by known methods.

The excipients used may be, for example, (1) inert diluents such as calcium carbonate, lactose, calcium phosphate or sodium phosphate; (2) granulating and disintegrating agents such as corn starch, potato starch or alginic acid; (3) binding agents such as gum tragacanth, corn starch, gelatin or acacia, and (4) lubricating agents such as magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monostearate or glyceryl distearate may be employed.

In some cases, formulations for oral use may be in the form of hard gelatin capsules wherein the compounds are mixed with an inert solid diluent, for example, calcium carbonate, calcium phosphate or kaolin. They may also be in the form of soft gelatin capsules wherein the compounds are mixed with water or an oil medium, for example, peanut oil, liquid paraffin, or olive oil.

The pharmaceutical compositions may also be in the form of a sterile injectable suspension. Suspensions may be formulated according to known methods using suitable dispersing or wetting agents and suspending agents. The sterile injectable preparations may also be sterile injectable solutions or suspensions in a non-toxic parenterally-acceptable diluent or solvent, for example, as a solution in 1,3-butanediol. Sterile, fixed oils are conventionally employed as a solvent or suspending medium. For this purpose any bland fixed oil may be employed including synthetic mono- or diglycerides, fatty acids (including oleic acid), naturally occurring vegetable oils like sesame oil, coconut oil, peanut oil, cottonseed oil, etc., or synthetic fatty vehicles like ethyl oleate or the like. Buffers, preservatives, antioxidants, and the like can be incorporated as required.

Compounds described herein may also be administered in the form of suppositories for rectal administration. These compositions may be prepared by mixing the compounds with a suitable non-irritating excipient, such as cocoa butter, synthetic glyceride esters of polyethylene glycols, which are solid at ordinary temperatures, but liquefy and/or dissolve in the rectal cavity to release the drug.

The compounds described herein can also be administered as an ophthalmically acceptable formulation or composition. A liquid which is ophthalmically acceptable is formulated such that it can be administered topically to the eye. The comfort should be maximized as much as possible, although sometimes formulation considerations (e.g. stability) may necessitate less than optimal comfort. In the case that comfort cannot be maximized, the liquid should be formulated such that the liquid is tolerable to the patient for topical ophthalmic use. Additionally, an ophthalmically acceptable liquid should either be packaged for single use, or contain a preservative to prevent contamination over multiple uses. For ophthalmic application, solutions or medicaments are often prepared using a physiological saline solution as a major vehicle. Ophthalmic solutions should preferably be maintained at a comfortable pH with an appropriate buffer system. The formulations may also contain conventional, pharmaceutically acceptable preservatives, stabilizers and surfactants.

Preservatives that may be used in ophthalmic compositions described herein include, but are not limited to, benzalkonium chloride, chlorobutanol, thimerosal, phenylmercuric acetate and phenylmercuric nitrate. A useful surfactant is, for example, Tween 80. Likewise, various useful vehicles may be used in the ophthalmic preparations described herein. These vehicles include, but are not limited to, polyvinyl alcohol, povidone, hydroxypropyl methyl cellulose, poloxamers, carboxymethyl cellulose, hydroxyethyl cellulose and purified water.

Tonicity adjustors may be added as needed or convenient. They include, but are not limited to, salts, particularly sodium chloride, potassium chloride, mannitol and glycerin, or any other suitable ophthalmically acceptable tonicity adjustor.

Various buffers and means for adjusting pH may be used so long as the resulting preparation is ophthalmically acceptable. Accordingly, buffers include acetate buffers, citrate buffers, phosphate buffers and borate buffers. Acids or bases may be used to adjust the pH of these formulations as needed.

In one example embodiment, an ophthalmic composition as described herein may have ingredients used in the following amounts listed in **Table 1.**

**Table 1**

| **Ingredient** | **Amount (% w/v)** |
|---|---|
| active ingredient | about 0.001-5 |
| preservative | 0-0.10 |
| vehicle | 0-40 |
| tonicity adjustor | 1-10 |
| buffer | 0.01-10 |
| pH adjustor | q.s. pH 4.5-7.5 |
| antioxidant | as needed |
| surfactant | as needed |
| purified water | as needed to make 100% |

In other embodiments, the ophthalmically acceptable liquid can be formulated for intraocular injection. The compounds described herein can be formulated as a liquid, gel paste, or the like for intraocular injection. Further, the compounds can be formulated into sustained release or controlled release intraocular implants comprising biodegradable polymers such as polylactic acid, poly glycolic acid, combinations thereof and the like.

Since individual subjects may present a wide variation in severity of symptoms and each composition has its unique therapeutic characteristics, the precise mode of administration and dosage employed for each subject is left to the discretion of the practitioner.

The present invention concerns also a process for preparing the compounds having Formula I.

The synthetic scheme set forth below, illustrates how compounds according to the invention can be made. Those skilled in the art will be able to routinely modify and/or adapt the following scheme to synthesize any compounds of the invention covered by formula I.

The N-(2,3-substituted phenyl)-3,4-dihydro-2H-pyrrol-5-amines of the invention were obtained according to the following general schemes. The primary amines are commercially available. In Scheme 1 the primary amine reacted with pyrrolidin-2-one in the presence of phosphoryl chloride to form the desired N-(2,3-substituted phenyl)-3,4-dihydro-2H-pyrrol-5-amine. The solvent for this reaction was toluene.

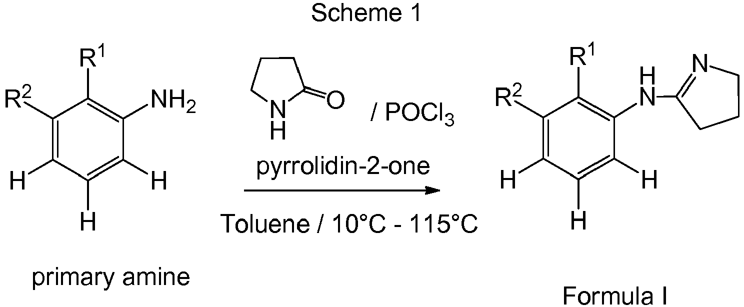

### Experimental details

The following examples are for illustrative purposes only and are not intended, nor should they be construed as limiting the invention in any manner. Those skilled in the art will appreciate that variations and modifications of the following examples can be made without exceeding the spirit or scope of the invention.

The IUPAC names of the compounds mentioned in the examples were generated with ACD version 8.

Unless specified otherwise in the examples, characterization of the compounds is performed according to the following methods:

NMR spectra are recorded on 300 MHz Varian and acquired at room temperature. Chemical shifts are given in p.p.m. referenced either to internal Tetramethylsilane or to the residual solvent signal.

All the reagents, solvents, catalysts for which the synthesis is not described are purchased from chemical vendors such as Sigma Aldrich, Fluka, Lancaster.

Usually the compounds of the invention were purified by flash column chromatography using as solvent system: 7N NH₃ in MeOH/DCM.

The following abbreviations are used in the examples:
- POCl₃: phosphoryl chloride
- DCM: dichloromethane
- NaOH: sodium hydroxyde
- MeOH: methanol
- CD₃OD: deuterated methanol
- NH₃: ammonia
- Na₂SO₄: sodium sulfate
- CD₃COOD: deuterated acetic acid

### Example 1

### N-(2-bromo-3-methyl-phenyl)-3,4-dihydro-2H-pyrrol-5-amine

### Compound 1

A solution of POCl₃ in 8 mL of toluene was added slowly to a solution of 2-pyrrolidinone in 8 mL of toluene at 10°C. Then, the reaction was stirred at room temperature for about 3 hours. A solution of 2-bromo-3-methyl-aniline in 8 mL of toluene was added, and the mixture was heated to reflux overnight. The refluxed mixture was cooled to room temperature, and the toluene layer was decanted. The residue was dissolved in aqueous water and dichloromethane (DCM). Aqueous 5N NaOH was used to adjust the pH to alkaline. Two layers were separated, and the organic layer was washed with water and brine, dried over Na₂SO₄ and concentrated under reduced vacuum. Flash column chromatography (2-4% 7N NH₃ in MeOH/DCM) yielded the title compound.
¹H NMR (300 MHz, CD₃OD) δ ppm 1.98 - 2.14 (m, 2 H) 2.39 (s, 3 H) 2.51 (t, *J*=8.06 Hz, 2 H) 3.38 (t, *J*=6.74 Hz, 2 H) 6.80 (d, *J*=7.33 Hz, 1 H) 6.95 (d, *J*=7.33 Hz, 1 H) 7.13 (t, *J*=7.62 Hz, 1 H).

Compounds 2 through 10 were prepared in a similar manner to the method described in Example 1 for Compound 1. The respective primary amines are commercially available. The results obtained are tabulated below in **Table 2.**

**Table 2**

| **Compound number** | **IUPAC name** | **¹H NMR (Solvent) δ ppm** |
|---|---|---|
| **2** | **N-(3-chloro-2-methoxyphenyl)-3,4-dihydro-2H-pyrrol-5-amine** | (CD₃OD) 2.22 - 2.40 (m, 2 H) 3.08 (t, *J*=8.06 Hz, 2 H) 3.72 (t, *J*=7.18 Hz, 2 H) 3.86 (s, 3 H) 6.67 (s, 2 H) 7.23 (t, *J*=7.92 Hz, 1 H) 7.32 (dd, *J*=7.92, 1.76 Hz, 1 H) 7.51 (dd, *J*=7.92, 1.76 Hz, 1 H) |
| **3** | **N-(3-chloro-2-methylphenyl)-3,4-dihydro-2H-pyrrol-5-amine** | (CDCl₃ with trace amount of CD₃COOD) 2.13 (m, 2H) 2.35(s, 3H) 2.58(t, J=7.80Hz, 2H) 3.80(t, J=7.11 Hz, 2H) 7.03(d, J=7.81 Hz, 1H) 7.15(t, J=8.07Hz, 1 H) 7.01 (d, J=8.07Hz, 1H) |
| 4 | **N-(2-chloro-3-methylphenyl)-3,4-dihydro-2H-pyrrol-5-amine** | (CD₃OD) 2.21 - 2.38 (m, 2 H) 2.46 (s, 3 H) 3.07 (t, *J*=8.06 Hz, 2 H) 3.70 (t, *J*=7.18 Hz, 2 H) 7.24 - 7.49 (m, 3 H) |
| 5 | **N-(2-methoxy-3-methylphenyl)-3,4-dihydro-2H-pyrrol-5-amine** | (CD₃OD) 2.22 - 2.33 (m, 2 H) 2.34 (s, 3 H) 3.03 - 3.18 (m, 2 H) 3.65 - 3.74 (m, 2 H) 3.75 (s, 3 H) 7.09 - 7.22 (m, 2 H) 7.30 (s, 1 H) |
| 6 | **N-(3-chloro-2-fluorophenyl)-3,4-dihydro-2H-pyrrol-5-amine** | (CDCl₃ with trace amount of CD₃COOD) 2.17 (m, 2H) 2.67(t, J=8.21 Hz, 2H) 3.76(t, J=7.04Hz, 2H) 7.14 (m, 2H) 7.32(m. 1H) |
| 7 | **N-(2-bromo-3-methoxyphenyl)-3,4-dihydro-2H-pyrrol-5-amine** | (CD₃OD) 2.00 - 2.11 (m, 2 H) 2.51 (t, *J*=7.82 Hz, 2 H) 3.38 (t, *J*=6.85 Hz, 2 H) 3.85 (s, 3 H) 6.60 (d, *J*=7.82 Hz, 1 H) 6.71 (d, *J*=8.31 Hz, 1 H) 7.20 (t, *J*=8.07 Hz, 1 H) |
| 8 | **N-(3-methoxy-2-methylphenyl)-3,4-dihydro-2H-pyrrol-5-amine** | (CD₃OD) 2.14 (s, 3 H) 2.21 - 2.39 (m, 2 H) 3.14 (t, *J*=8.06 Hz, 2 H) 3.68 (t, *J*=7.18 Hz, 2 H) 3.88 (s, 3 H) 6.92 (d, *J*=7.92 Hz, 1 H) 7.04 (d, *J*=8.50 Hz, 1 H) 7.32 (t, *J*=8.21 Hz, 1 H) |
| 9 | **N-(3-fluoro-2-methylphenyl)-3,4-dihydro-2H-pyrrol-5-amine** | (CD₃OD) 2.20 (d, *J*=2.05 Hz, 3 H) 2.22 - 2.34 (m, 2 H) 3.06 (t, *J*=8.06 Hz, 2 H) 3.67 (t, *J*=7.18 Hz, 2 H) 7.08 - 7.22 (m, 2 H) 7.28 - 7.41 (m, 1 H) |
| 10 | **N-(3-bromo-2-methylphenyl)-3,4-dihydro-2H-pyrrol-5-amine** | (CD₃OD) 2.20 - 2.35 (m, 2 H) 2.36 (s, 3 H) 3.07 (t, *J*=7.92 Hz, 2 H) 3.67 (t, *J*=7.18 Hz, 2 H) 7.19 - 7.34 (m, 2 H) 7.68 (dd, *J*=7.92, 1.47 Hz, 1 H) |

### Example 2

### In Vitro Activity

Compounds described herein were synthesized and tested for alpha adrenergic activity using a Fluorometric Imaging plate Reader (FLIPR) assay (Princen et al., 2003, Cytometry Part A. 51, pp. 35-45). Cells expressing the receptor of interest (alpha-2C) and a G-protein (Gqi5 or G16) are loaded with fluo-4, a calcium sensitive dye. After removal of excess dye by washing, the cells are placed in the FLIPR TETRA instrument. Baseline fluorescence readings are taken prior to addition of the compounds. Agonists will trigger the receptor to interact with the G-protein, leading to an increase in intracellular calcium. The increase in intracellular calcium causes an increase in the fluorescence of the cells, due to the presence of fluo-4. This fluorescence increase is recorded by the FLIPR TETRA. After the calcium transient signal has decreased towards baseline, the standard agonist norepinephrine is added. If the compound is an antagonist, an initial calcium signal will not be generated and the antagonist will prevent the generation of a calcium signal from norepinephrine. The level of fluorescence is compared to that of norepinephrine, and the EC₅₀ of the compound determined by curve fitting. The compound's activity is expressed as EC₅₀ and its relative efficacy compared to a standard full agonist (see Table 3 below). The compounds described herein activate alpha adrenergic receptors.

**Table 3**

| Potency (*n*M); efficacy (EC₅₀) | | |
|---|---|---|
| **Compound number** | **IUPAC name** | **Alpha 2C** |
| **1** | N-(2-bromo-3-methyl-phenyl)-3,4-dihydro-2H-pyrrol-5-amine | 663 (1) |
| **2** | N-(3-chloro-2-methoxyphenyl)-3,4-dihydro-2H-pyrrol-5-amine | 975 (1) |
| **3** | N-(3-chloro-2-methylphenyl)-3,4-dihydro-2H-pyrrol-5-amine | 71 (1) |
| **4** | N-(2-chloro-3-methylphenyl)-3,4-dihydro-2H-pyrrol-5-amine | 635 (1) |
| **5** | N-(2-methoxy-3-methylphenyl)-3,4-dihydro-2H-pyrrol-5-amine | 243 (1) |
| **6** | N-(3-chloro-2-fluorophenyl)-3,4-dihydro-2H-pyrrol-5-amine | 26 (1) |
| **7** | N-(2-bromo-3-methoxyphenyl)-3,4-dihydro-2H-pyrrol-5-amine | 32 (1) |
| **8** | N-(3-methoxy-2-methylphenyl)-3,4-dihydro-2H-pyrrol-5-amine | 186 (1) |
| **9** | N-(3-fluoro-2-methylphenyl)-3,4-dihydro-2H-pyrrol-5-amine | 756 (1) |
| **10** | N-(3-bromo-2-methylphenyl)-3,4-dihydro-2H-pyrrol-5-amine | 288 (1) |
| **11** | N-(2-chlorophenyl)-3,4-dihydro-2H-pyrrol-5-amine | 709 (1) |
| **12** | N-(2,6-dichlorophenyl)-3,4-dihydro-2H-pyrrol-5-amine | 44 (1) |
| **13** | N-(2-methylphenyl)-3,4-dihydro-2H-pyrrol-5-amine | 743 (1) |
| **14** | N-[2-(trifluoromethyl)phenyl]-3,4-dihydro-2H-pyrrol-5-amine | 796 (1) |
| **15** | N-(2,6-dimethylphenyl)-3,4-dihydro-2H-pyrrol-5-amine | 348 (1) |
| **16** | N-(2,3-dimethylphenyl)-3,4-dihydro-2H-pyrrol-5-amine | 118 (1) |

## Claims

1. A compound represented by the following Formula I or a pharmaceutically-acceptable salt, hydrate, solvate or tautomer thereof: 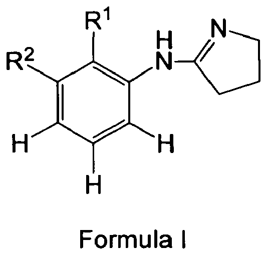 wherein:
R¹ is selected from methoxy, methyl, chloro, fluoro and bromo; and
R² is selected from methoxy, methyl, chloro, fluoro and bromo;
with the proviso that R¹ and R² are not both methyl.

2. A compound or a derivative thereof according to Claim 1, which is selected from the following compounds:
N-(2-bromo-3-methyl-phenyl)-3,4-dihydro-2H-pyrrol-5-amine;
N-(3-chloro-2-methoxyphenyl)-3,4-dihydro-2H-pyrrol-5-amine;
N-(3-chloro-2-methylphenyl)-3,4-dihydro-2H-pyrrol-5-amine;
N-(2-chloro-3-methylphenyl)-3,4-dihydro-2H-pyrrol-5-amine;
N-(2-methoxy-3-methylphenyl)-3,4-dihydro-2H-pyrrol-5-amine;
N-(3-chloro-2-fluorophenyl)-3,4-dihydro-2H-pyrrol-5-amine;
N-(2-bromo-3-methoxyphenyl)-3,4-dihydro-2H-pyrrol-5-amine;
N-(3-methoxy-2-methylphenyl)-3,4-dihydro-2H-pyrrol-5-amine;
N-(3-fluoro-2-methylphenyl)-3,4-dihydro-2H-pyrrol-5-amine;
N-(3-bromo-2-methylphenyl)-3,4-dihydro-2H-pyrrol-5-amine.

3. A pharmaceutical composition comprising a compound or a derivative thereof as defined in Claim 1.

4. A pharmaceutical composition according to Claim 3, which comprises at least one of the following compounds:
N-(2-bromo-3-methyl-phenyl)-3,4-dihydro-2H-pyrrol-5-amine;
N-(3-chloro-2-methoxyphenyl)-3,4-dihydro-2H-pyrrol-5-amine;
N-(3-chloro-2-methylphenyl)-3,4-dihydro-2H-pyrrol-5-amine;
N-(2-chloro-3-methylphenyl)-3,4-dihydro-2H-pyrrol-5-amine;
N-(2-methoxy-3-methylphenyl)-3,4-dihydro-2H-pyrrol-5-amine;
N-(3-chloro-2-fluorophenyl)-3,4-dihydro-2H-pyrrol-5-amine;
N-(2-bromo-3-methoxyphenyl)-3,4-dihydro-2H-pyrrol-5-amine;
N-(3-methoxy-2-methylphenyl)-3,4-dihydro-2H-pyrrol-5-amine;
N-(3-fluoro-2-methylphenyl)-3,4-dihydro-2H-pyrrol-5-amine;
N-(3-bromo-2-methylphenyl)-3,4-dihydro-2H-pyrrol-5-amine.

5. A compound or a derivative thereof as defined in Claim 1 for use in the treatment of a disorder associated with selective subtype modulation of the alpha 2C adrenergic receptor.

6. A compound or a derivative thereof for use according to Claim 5, which is selected from the following compounds:
N-(2-bromo-3-methyl-phenyl)-3,4-dihydro-2H-pyrrol-5-amine;
N-(3-chloro-2-methoxyphenyl)-3,4-dihydro-2H-pyrrol-5-amine;
N-(3-chloro-2-methylphenyl)-3,4-dihydro-2H-pyrrol-5-amine;
N-(2-chloro-3-methylphenyl)-3,4-dihydro-2H-pyrrol-5-amine;
N-(2-methoxy-3-methylphenyl)-3,4-dihydro-2H-pyrrol-5-amine;
N-(3-chloro-2-fluorophenyl)-3,4-dihydro-2H-pyrrol-5-amine;
N-(2-bromo-3-methoxyphenyl)-3,4-dihydro-2H-pyrrol-5-amine;
N-(3-methoxy-2-methylphenyl)-3,4-dihydro-2H-pyrrol-5-amine;
N-(3-fluoro-2-methylphenyl)-3,4-dihydro-2H-pyrrol-5-amine;
N-(3-bromo-2-methylphenyl)-3,4-dihydro-2H-pyrrol-5-amine.

7. A compound or a derivative thereof for use according to Claim 5, wherein the disorder is nasal congestion.

8. A compound or a derivative thereof for use according to Claim 5, wherein the disorder is selected from pain, cardiac ischemia, migraine, dermatological conditions, skin erythema (redness) and inflammation, rosacea, acne, psoriasis, depression and schizophrenia.

## Patentansprüche

1. Verbindung der nachstehenden Formel (I) oder ein pharmazeutisch annehmbares Salz, Hydrat, Solvat oder Tautomer davon: 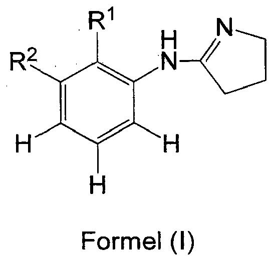 worin:
R¹ aus Methoxy, Methyl, Chlor, Fluor und Brom ausgewählt ist; und
R² aus Methoxy, Methyl, Chlor, Fluor und Brom ausgewählt ist;
mit der Massgabe, dass R¹ und R² nicht beide Methyl sind.

2. Verbindung oder ein Derivat davon gemäss Anspruch 1, die aus den nachstehenden Verbindungen ausgewählt ist:
N-(2-Brom-3-methyl-phenyl)-3,4-dihydro-2H-pyrrol-5-amin;
N-(3-Chlor-2-methoxyphenyl)-3,4-dihydro-2H-pyrrol-5-amin;
N-(3-Chlor-2-methylphenyl)-3,4-dihydro-2H-pyrrol-5-amin;
N-(2-Chlor-3-methylphenyl)-3,4-dihydro-2H-pyrrol-5-amin;
N-(2-Methoxy-3-methylphenyl)-3,4-dihydro-2H-pyrrol-5-amin;
N-(3-Chlor-2-fluorphenyl)-3,4-dihydro-2H-pyrrol-5-amin;
N-(2-Brom-3-methoxyphenyl)-3,4-dihydro-2H-pyrrol-5-amin;
N-(3-Methoxy-2-methylphenyl)-3,4-dihydro-2H-pyrrol-5-amin;
N-(3-Fluor-2-methylphenyl)-3,4-dihydro-2H-pyrrol-5-amin;
N-(3-Brom-2-methylphenyl)-3,4-dihydro-2H-pyrrol-5-amin.

3. Pharmazeutische Zusammensetzung, die eine Verbindung oder ein Derivat davon, wie in Anspruch 1 definiert, umfasst.

4. Pharmazeutische Zusammensetzung gemäss Anspruch 3, die mindestens eine der nachstehenden Verbindungen umfasst:
N-(2-Brom-3-methyl-phenyl)-3,4-dihydro-2H-pyrrol-5-amin;
N-(3-Chlor-2-methoxyphenyl)-3,4-dihydro-2H-pyrrol-5-amin;
N-(3-Chlor-2-methylphenyl)-3,4-dihydro-2H-pyrrol-5-amin;
N-(2-Chlor-3-methylphenyl)-3,4-dihydro-2H-pyrrol-5-amin;
N-(2-Methoxy-3-methylphenyl)-3,4-dihydro-2H-pyrrol-5-amin;
N-(3-Chlor-2-fluorphenyl)-3,4-dihydro-2H-pyrrol-5-amin;
N-(2-Brom-3-methoxyphenyl)-3,4-dihydro-2H-pyrrol-5-amin;
N-(3-Methoxy-2-methylphenyl)-3,4-dihydro-2H-pyrrol-5-amin;
N-(3-Fluor-2-methylphenyl)-3,4-dihydro-2H-pyrrol-5-amin;
N-(3-Brom-2-methylphenyl)-3,4-dihydro-2H-pyrrol-5-amin.

5. Verbindung oder ein Derivat davon, wie in Anspruch 1 definiert, zur Verwendung bei der Behandlung einer Störung, die mit einer selektiven Modulation des Subtyps von alpha-2C-adrenergem Rezeptor in Verbindung steht.

6. Verbindung oder ein Derivat davon zur Verwendung gemäss Anspruch 5, die aus den nachstehenden Verbindungen ausgewählt ist:
N-(2-Brom-3-methyl-phenyl)-3,4-dihydro-2H-pyrrol-5-amin;
N-(3-Chlor-2-methoxyphenyl)-3,4-dihydro-2H-pyrrol-5-amin;
N-(3-Chlor-2-methylphenyl)-3,4-dihydro-2H-pyrrol-5-amin;
N-(2-Chlor-3-methylphenyl)-3,4-dihydro-2H-pyrrol-5-amin;
N-(2-Methoxy-3-methylphenyl)-3,4-dihydro-2H-pyrrol-5-amin;
N-(3-Chlor-2-fluorphenyl)-3,4-dihydro-2H-pyrrol-5-amin;
N-(2-Brom-3-methoxyphenyl)-3,4-dihydro-2H-pyrrol-5-amin;
N-(3-Methoxy-2-methylphenyl)-3,4-dihydro-2H-pyrrol-5-amin;
N-(3-Fluor-2-methylphenyl)-3,4-dihydro-2H-pyrrol-5-amin;
N-(3-Brom-2-methylphenyl)-3,4-dihydro-2H-pyrrol-5-amin.

7. Verbindung oder ein Derivat davon zur Verwendung gemäss Anspruch 5, wobei die Störung eine verstopfte Nase ist.

8. Verbindung oder ein Derivat davon zur Verwendung gemäss Anspruch 5, wobei die Störung aus Schmerzen, kardialer Ischämie, Migräne, dermatologischen Erkrankungen, Hauterythem (Rötung) und Entzündungen, Rosacea, Akne, Psoriasis, Depression und Schizophrenie ausgewählt ist.

## Revendications

1. Composé représenté par la formule I suivante ou un sel, hydrate, solvate ou tautomère pharmaceutiquement acceptable de celui-ci : 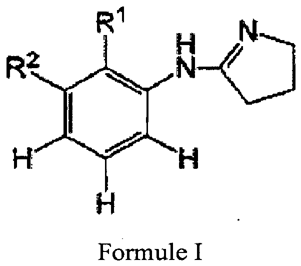 dans laquelle :
R¹ est choisi parmi un méthoxy, un méthyle, un chloro, un fluoro et un bromo ; et
R² est choisi parmi un méthoxy, un méthyle, un chloro, un fluoro et un bromo ;
à condition que R¹ et R² ne soient pas tous les deux des méthyles.

2. Composé ou dérivé de celui-ci selon la revendication 1, qui est choisi parmi les composés suivants :
la N-(2-bromo-3-méthyl-phényl)-3,4-dihydro-2H-pyrrol-5-amine ;
la N-(3-chloro-2-méthoxyphényl)-3,4-dihydro-2H-pyrrol-5-amine ;
la N-(3-chloro-2-méthylphényl)-3,4-dihydro-2H-pyrrol-5-amine ;
la N-(2-chloro-3-méthylphényl)-3,4-dihydro-2H-pyrrol-5-amine ;
la N-(2-méthoxy-3-méthylphényl)-3,4-dihydro-2H-pyrrol-5-amine ;
la N-(3-chloro-2-fluorophényl)-3,4-dihydro-2H-pyrrol-5-amine ;
la N-(2-bromo-3-méthoxyphényl)-3,4-dihydro-2H-pyrrol-5-amine ;
la N-(3-méthoxy-2-méthylphényl)-3,4-dihydro-2H-pyrrol-5-amine ;
la N-(3-fluoro-2-méthylphényl)-3,4-dihydro-2H-pyrrol-5-amine ;
la N-(3-bromo-2-méthylphényl)-3,4-dihydro-2H-pyrrol-5-amine.

3. Composition pharmaceutique comprenant un composé ou un dérivé de celui-ci tel que défini dans la revendication 1.

4. Composition pharmaceutique selon la revendication 3, qui comprend au moins un des composés suivants :
la N-(2-bromo-3-méthyl-phényl)-3,4-dihydro-2H-pyrrol-5-amine ;
la N-(3-chloro-2-méthoxyphényl)-3,4-dihydro-2H-pyrrol-5-amine ;
la N-(3-chloro-2-méthylphényl)-3,4-dihydro-2H-pyrrol-5-amine ;
la N-(2-chloro-3-méthylphényl)-3,4-dihydro-2H-pyrrol-5-amine ;
la N-(2-méthoxy-3-méthylphényl)-3,4-dihydro-2H-pyrrol-5-amine ;
la N-(3-chloro-2-fluorophényl)-3,4-dihydro-2H-pyrrol-5-amine ;
la N-(2-bromo-3-méthoxyphényl)-3,4-dihydro-2H-pyrrol-5-amine ;
la N-(3-méthoxy-2-méthylphényl)-3,4-dihydro-2H-pyrrol-5-amine ;
la N-(3-fluoro-2-méthylphényl)-3,4-dihydro-2H-pyrrol-5-amine ;
la N-(3-bromo-2-méthylphényl)-3,4-dihydro-2H-pyrrol-5-amine.

5. Composé ou dérivé de celui-ci tel que défini dans la revendication 1, pour une utilisation dans le traitement d'un trouble associé à la modulation sélective de sous-type du récepteur adrénergique alpha 2C.

6. Composé ou dérivé de celui-ci pour une utilisation selon la revendication 5, qui est choisi parmi les composés suivants :
la N-(2-bromo-3-méthyl-phényl)-3,4-dihydro-2H-pyrrol-5-amine ;
la N-(3-chloro-2-méthoxyphényl)-3,4-dihydro-2H-pyrrol-5-amine ;
la N-(3-chloro-2-méthylphényl)-3,4-dihydro-2H-pyrrol-5-amine ;
la N-(2-chloro-3-méthylphényl)-3,4-dihydro-2H-pyrrol-5-amine ;
la N-(2-méthoxy-3-méthylphényl)-3,4-dihydro-2H-pyrrol-5-amine ;
la N-(3-chloro-2-fluorophényl)-3,4-dihydro-2H-pyrrol-5-amine ;
la N-(2-bromo-3-méthoxyphényl)-3,4-dihydro-2H-pyrrol-5-amine ;
la N-(3-méthoxy-2-méthylphényl)-3,4-dihydro-2H-pyrrol-5-amine ;
la N-(3-fluoro-2-méthylphényl)-3,4-dihydro-2H-pyrrol-5-amine ;
la N-(3-bromo-2-méthylphényl)-3,4-dihydro-2H-pyrrol-5-amine.

7. Composé ou dérivé de celui-ci pour une utilisation selon la revendication 5, dans lequel le trouble est une congestion nasale.

8. Composé ou dérivé de celui-ci pour une utilisation selon la revendication 5, dans lequel le trouble est choisi parmi la douleur, l'ischémie cardiaque, la migraine, les affections dermatologiques, l'érythème cutané (rougeur) et l'inflammation cutanée, la couperose, l'acné, le psoriasis, la dépression et la schizophrénie.
